# EUROPEAN PATENT APPLICATION

(11) **EP 4 837 975 A1**
(43) Date of publication of application: **11.11.2026**
(21) Application number: 26176537.4
(22) Date of filing: 05.05.2026
(51) Int. Cl.: C12Q 1/6886

(54) **QPCR KIT FOR SIMULTANEOUS DETECTION OF 10 DIFFERENT TARGET GENE METHYLATION IN BOTH STOOL AND SERUM SAMPLES FOR EARLY COLORECTAL CANCER DIAGNOSIS**

(30) Priority: 08.05.2025 TR 2025005749
(71) Applicant: Eryigit Endustriyel Makina Ve Tibbi Cihazlar Imalat Ithalat Ihracat Insaat Ticaret Anonim Sirketi, 06378 Ankara (TR)
(72) Inventor: SAKALAR, Cagri, ANKARA (TR); DEDE, Serap, ANKARA (TR); CUBUKCI, Gamze, ANKARA (TR)
(74) Representative: Metida

(57) **Abstract**

The invention was developed to identify 10 different methylated gene regions and 1 unmethylated control region in DNA samples purified from stool and serum samples of normal individuals and colorectal cancer (CRC) patients undergoing screening.

This kit includes a bisulfite step for use in methylation analysis of purified DNA.

Real-time polymerase chain reaction (qPCR) reactions were run simultaneously for 10 target genes in stool samples and 5 target genes in serum specimens from the same patient.

Using specific primers for each target gene region and probe sets labeled with different fluorophores, the following were detected in 11 single reactions for the 10 target gene regions in the stool samples.

In the reactions for stool samples, it identifies and determines the methylation status of methylated CpG islands belonging to the BMP3, NDRG4, SEPTIN9, ITGA4, SFRP2, SDC2 MGMT, TFPI2, ADHFE1, and HOXA2 genes, and the unmethylated CpG island of BMP3 as a control in 11 single reactions for each target gene region.

It identifies and determines the methylation status of methylated CpG islands belonging to the SEPTIN9, SFRP2, SDC2, TFPI2, and HOXA2 genes, and the unmethylated CpG island of BMP3 as a control, in 6 single reactions for 5 target gene regions in serum specimens.

The invention is related to "Quantitative Methylation Detection of 10 Different Target Gene CpG Sites in Stool and Serum Samples for the Early Diagnosis of Colorectal Cancer Using Primer and Probe Sets." with the forementioned specifications.

## Description

The invention was developed to identify 10 different methylated gene regions and 1 unmethylated control region in DNA samples purified from stool and serum samples of normal individuals and colorectal cancer (CRC) patients undergoing screening.

This kit includes a bisulfite step for use in methylation analysis of purified DNA.

Real-time polymerase chain reaction (qPCR) reactions were run simultaneously for 10 target genes in stool samples and 5 target genes in serum samples from the same patient.

Using specific primers for each target gene region and probe sets labeled with different fluorophores, the kit also tested the methylation status of methylated CpG islands belonging to the SFRP2, SDC2, MGMT, TFPI2, ADHFE1, HOXA2, BMP3, NDRG4, SEPTIN9, and ITGA4 genes and the unmethylated CpG island of BMP3 as a control in 11 single reactions for the 10 target gene regions in stool samples was identified.

The methylation status of methylated CpG islands belonging to the SEPTIN9, SFRP2, SDC2, TFPI2, and HOXA2 genes, and the unmethylated CpG island of BMP3 as a control, was identified in 6 single reactions for 5 target gene regions in serum samples.

The results were analyzed to determine whether a sample was considered positive if it was positive for (TFPI2 and SEPTIN9) or ITGA4 or SDC2, or MGMT in stool samples, or if it was positive for (TFPI2 and SFRP2), or SDC2 in serum samples.

This article is about "Simultaneous Methylation Assessment of 10 Different Target Gene CpG Sites in Stool and Serum Samples for Early Diagnosis of Colorectal Cancer.".

### State of the Art:

Epigenetic modifications include modifications that do not alter genetic structure but affect gene expression. The most well-studied epigenetic modification is DNA methylation at the fifth cytosine position in CpG dinucleotides. DNA methylation is an epigenetic mechanism that regulates gene expression at many stages in the cell and embryonic development. DNA methylation is observed in specific regions of the human genome, called CpG islands, which are rich in CpG dinucleotides. DNA methylation changes in cancer have been known for many years. Hypermethylation of CpG islands of tumor suppressor genes is prominent in cancer. Changes in DNA methylation are thought to be useful biomarkers for diagnosis, prognosis, and determining treatment response. One of the most important ways to reduce mortality from CRC is early diagnosis. Early diagnosis through colonoscopy reduces the risk of death by up to 67%. However, because colonoscopy is not always a convenient method, faster, less invasive, more sensitive, and more practical methods are needed. Fecal immunochemical tests (FIT), fecal occult blood tests, or DNA-based tests are emerging as alternative early diagnosis tests. DNA methylation-based tests have great potential as tests that can enable early diagnosis from blood or stool. Studies are currently investigating DNA methylation-based biomarkers in CRC, and these studies use qPCR to detect DNA methylation. Studies have investigated methylation biomarkers in biological samples such as plasma, stool, and urine. These studies have identified frequently identified biomarkers such as the BMP3, NDRG4, and SEPT9 genes and their promoter regions, which have different functions related to tumor progression. Two FDA-approved kits are available for DNA methylation analysis in CRC diagnosis. One of these kits focuses on the methylation level of the SEPT9 gene in the blood. Clinical studies with the latest version, Epi proColon 2, have found sensitivity around 75-77%, while specificity has reached up to 96%. Another kit used in CRC diagnosis is the Cologuard test. It focuses on methylation in the BMP3 and NDRG4 gene regions, as well as KRAS mutations and fecal hemoglobin. Screening 252 CRC patients and 293 healthy participants yielded a sensitivity of 85% with a specificity of 90% for Cologuard. In 2014, a clinical screening study involving 9,989 participants demonstrated a sensitivity of 92% for colorectal cancer and 42% for precancerous lesions, while the specificity was 90%. Compared to the Cologuard panel, the FIT test was found to have lower sensitivity and higher specificity (74% and 96%). In addition, another test produced in China, approved by China and used in Europe is the Colosafe test. It focuses on the methylation of the SDC2 gene. This test has a sensitivity of approximately 85% for CRC.

Early diagnosis is crucial in colorectal cancer treatment. Methods such as fecal occult blood testing and colonoscopy are used for this purpose. Although fecal occult blood testing is a non-invasive method, its sensitivity and specificity are lower than those of molecular methods. While colonoscopy is an important method in CRC diagnosis, it requires specialist expertise, is invasive, and is less preferred by patients. In recent years, methylation-based molecular methods have gained importance in CRC diagnosis.

While kits for detection using stool or serum specimens are available worldwide, there is no diagnostic kit that analyzes both stool and serum simultaneously.

There is no study or product that simultaneously analyzes 10 target gene CpG regions in both stool and serum specimens.

### Purpose of the invention:

Conducting methylation analysis in 10 target gene regions in stool samples and methylation analysis in 5 target gene regions in serum specimens using a methylation-based qPCR method by analyzing both stool and serum simultaneously.

Achieving early diagnosis of colorectal cancer with high sensitivity and specificity by analyzing stool and serum results from the same individual.

For a sample to be considered positive, the stool samples must be positive for a combination of 5 genes (TFPI2 and SEPTIN9) or ITGA4 or SDC2, or MGMT, or a combination of 3 genes (TFPI2 and SFRP2), or SDC2 in serum specimens.

Using a methylation-based qPCR method, methylation of 10 gene regions in stool DNA and 5 gene regions in serum DNA will be detected from individuals. These methylation results will be analyzed to determine the risk of CRC and other cancers. The aim is to perform early diagnosis of precancerous lesions.

The feature of the invention is; DNA purified from stool and serum samples collected from individuals is used.

The test kit uses a combination of primer and probe sets.

Methylation is detected at CpG sites in 10 target genes.

It includes 11 single qPCR reactions in stool samples.

It includes 6 single qPCR reactions in serum samples.

Reactions performed in stool samples identify and determine the methylation status of methylated CpG islands belonging to the BMP3, NDRG4, SEPTIN9, ITGA4, SFRP2, SDC2, MGMT, TFPI2, ADHFE1, and HOXA2 genes, and the unmethylated CpG island of BMP3 as a control.

Reactions performed in serum specimens identify and determine the methylation status of methylated CpG islands belonging to the SEPTIN9, SFRP2, SDC2, TFPI2, and HOXA2 genes, and the unmethylated CpG island of BMP3 as a control. The method is to diagnose CRC by analyzing the results obtained from stool and serum samples together.

For a sample to be considered positive in the analysis of the results, the combination of five genes (TFPI2 and SEPTIN9) or ITGA4 or SDC2, or MGMT must be positive in the stool samples, or the combination of three genes (TFPI2 and SFRP2), or SDC2 must be positive in the serum specimens. The positivity of other genes increases the test score, increasing the likelihood that the test sample originates from a cancer patient.

These results were confirmed with clinical samples.

The figures prepared for a better understanding of the invention are as follows:
The explanation of the numbers in the figures is as follows:
1. Fluorescence Signal
2. Number of Cycles
3. qPCR Graph
4. Cancer Cell Lines
5. Patient Samples
6. Negative Control
7. True Positive Fraction
8. False Positive Fraction
9. ROC Curve

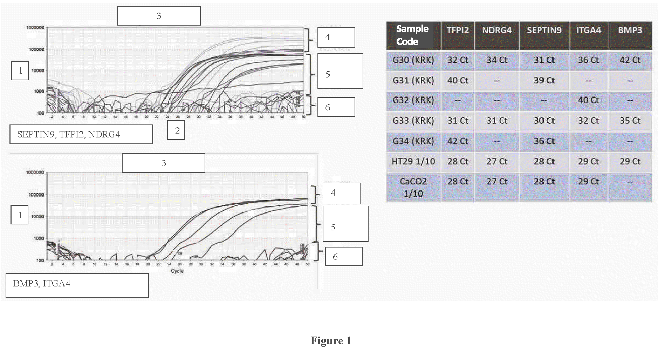

Figure 1. Stool samples from 5 CRC patients (5) were tested using a methylation-based qPCR kit for early diagnosis of CRC. The table on the right shows the 5 target gene regions (TFPI2, NDRG4, SEPTIN9, ITGA4, BMP3), patient codes (G30 to G34), and Ct values indicating the qPCR signal value. Results with a Ct value less than 40 are considered positive. HT-29 and CaCo-2, colorectal cancer cell lines (4), were used as positive controls. The graphs on the left show the amplification curves from the qPCR results (3). The Y-axis represents the fluorescence signal (1), and the X-axis represents the cycle number (2). The patient samples (5) representing the amplification curves are shown in the figure. CRC = Colorectal Cancer, NK = Negative Control (6).

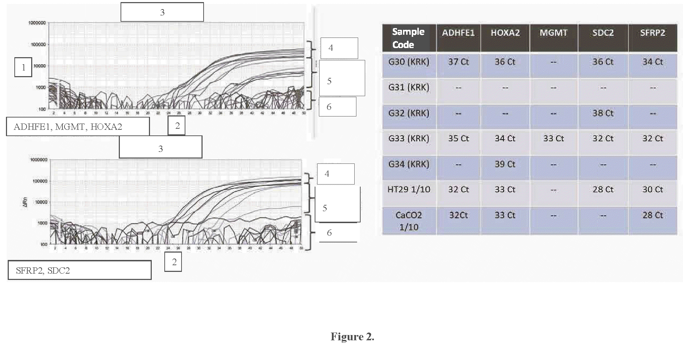

Figure 2. A methylation-based qPCR kit was used to test stool samples (5) from 5 CRC patients for early diagnosis of CRC. The table on the right shows the 5 target gene regions (ADHFE1, HOXA2, MGMT, SDC2, SFRP2), patient codes (G30 to G34), and Ct values indicating the qPCR signal value. Results with a Ct value less than 40 are considered positive. HT-29 and CaCo-2, colorectal cancer cell lines (4), were used as positive controls. The graphs on the left are the amplification curves seen as a result of qPCR (3). The Y-axis shows the fluorescence signal (1), and the X-axis shows the cycle count (2). The patient samples (5) to which the amplification curves belong are shown in the figure. CRC = Colorectal Cancer, NK = Negative Control (6).

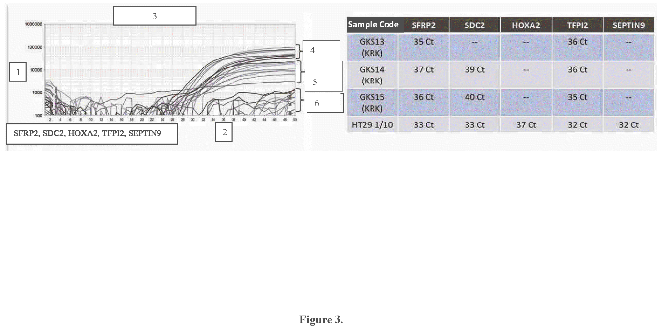

Figure 3. Serum specimens from three CRC patients (5) were tested using a methylation-based qPCR kit for early diagnosis of CRC. The table on the right shows the five target gene regions (SFRP2, SDC2, HOXA2, SEPTIN9, TFPI2), patient codes (GKS13, 14, and 15), and Ct values indicating the qPCR signal value. Results with a Ct value less than 40 are considered positive. HT-29, a colorectal cancer cell line (4), was used as a positive control. The graphs on the left are the amplification curves seen as a result of qPCR (3). The Y-axis shows the fluorescence signal (1), and the X-axis shows the cycle count (2). The patient samples (5) from which the amplification curves are shown are shown in the figure. CRC = Colorectal Cancer, NK = Negative Control (6).

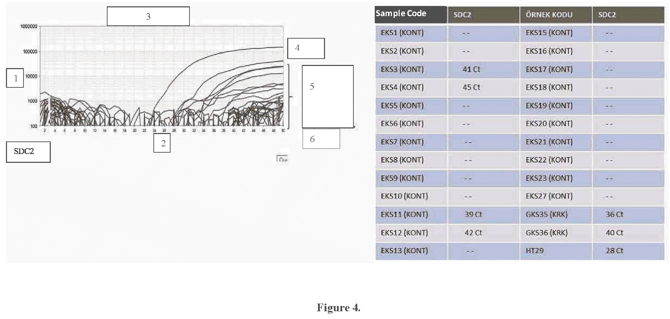

Figure 4. A methylation-based qPCR kit was used to test the early diagnosis of CRC using serum specimens (5) from 23 control individuals and 2 CRC patients. The table on the right shows the 5 target gene regions (SFRP2, SDC2, HOXA2, SEPTIN9, TFPI2), control and patient codes (EKS1-27, GKS35 and 36), and Ct values indicating the qPCR signal value. Results with a Ct value less than 40 are considered positive. HT-29, a colorectal cancer cell line (4), was used as a positive control. The graphs on the left show the amplification curves from the qPCR results (3). The Y-axis shows the fluorescence signal (1), and the X-axis shows the cycle number (2). The patient samples (5) from which the amplification curves are shown in the figure. CRC = Colorectal Cancer, NC = Negative Control (6).

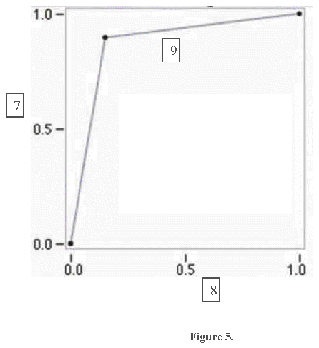

Figure 5. Stool and serum specimens from 48 CRC patients and 33 healthy controls were analyzed using the detection output. The results were included in the ROC analysis to generate the ROC curve (9). The X-axis shows the false positive fraction (8), and the Y-axis shows the true positive fraction (7). Both stool and serum results were evaluated together for each individual. TFPI2, SEPTIN9, ITGA4, SDC2, and MGMT were prioritized for cancer scoring in stool results, while SFRP2, TFPI2, and SDC2 were prioritized for serum results. Individuals evaluated as cancerous were given a score of 2, and individuals evaluated as cancer-negative were given a score of 1. According to this analysis, the sensitivity of the detection output was calculated as 89.6%, specificity as 84.8%, and the AUC (Area Under the Curve) value as 0.872.

**Storage conditions for the reaction mixture containing the primers and probes in the kit:** In addition to the primer and probe sets mentioned above, the reaction mixture contains reaction buffer, nucleotides, and DNA polymerase enzyme and is stored at -15°C/-25°C. Template DNA used as a positive control is also stored at -15°C/-25°C. Probes are light-sensitive, and therefore, the reaction mixtures are shipped in brown vials. Therefore, the reaction mixtures must be protected from light.

### Reaction setup:

For early diagnosis of CRC, both stool and serum specimens will be analyzed from the same individual. First, DNA will be isolated from these samples and bisulfite will be used. After this process, the bisulfite DNA will be used as the template DNA in the test output. For stool samples, 11 reactions will be set up: specific primer-probe sets will be used for methylated CpG islands belonging to the BMP3, NDRG4, SEPTIN9, ITGA4, SFRP2, SDC2, MGMT, TFPI2, ADHFE1, and HOXA2 genes, and the unmethylated CpG island of BMP3 as a control. For serum samples, 6 reactions will be set up: specific primer-probe sets will be used for methylated CpG islands belonging to the SEPTIN9, SFRP2, SDC2, TFPI2, and HOXA2 genes, and the unmethylated CpG island of BMP3 as a control. These reactions will be set up as a single-plex.
a. A negative control (NK) and a positive control (PK) are always used in each run. Gene synthesis of the TFPI2 final target region is used as a positive control.
b. The purpose of the negative control is to determine whether there is any nucleic acid contamination in the materials used. A negative control is mandatory to avoid false-positive results in samples used for early CRC diagnosis. Negative control samples without added nucleic acid must be free of amplification.
c. The TFPI2 synthetic gene region (or postive control DNA) provided in the kit should be added to the wells/tubes used as positive controls. The purpose of the positive control is to prevent false-negative results that may occur due to run-related errors. A positive control is mandatory to avoid false-negative results in samples used for early CRC diagnosis. The relevant amplification must be observed in the positive control samples.

### Plate design:

The wells of the plate/strip tubes to be used should contain 11 reactions for each sample (for the 11 target regions described above) for the stool sample and 6 reactions for the serum sample (for the 6 target regions described above), also positive control and negative control reactions are included, and the qPCR device software is entered in this way. An example plate design is given in Table 1 below (S1-G-R1: sample 1-stool-reaction 1, S1-G-R2: sample 1-stool-reaction 2, S1-G-R3: sample 1-stool-reaction 3, S1-G-R4: sample 1-stool-reaction 4, S1-G-R5: sample 1-stool-reaction 5, S1-G-R6: sample 1-stool-reaction 6, S1-G-R7: sample 1-stool-reaction 7, S1-G-R8: sample 1-stool-reaction 8, S1-G-R9: sample 1-stool-reaction 9, S1-G-R10: sample 1-stool-reaction 10, S1-G-R11: sample 1-stool-reaction 11, S1-S-R1: sample 1-serum-reaction 1, S1-S-R2: sample 1-serum-reaction 2, S1-S-R3: sample 1-serum-reaction 3, S1-S-R4: sample 1-serum-reaction 4, S1-S-R5: sample 1-serum-reaction 5, S1-S-R6: sample 1-serum-reaction 6, R1NK: Reaction 1 negative control, R2NK: Reaction 2 negative control, R3NK: Reaction 3 negative control, R4NK: Reaction 4 negative control, R5NK: Reaction 5 negative control, R6NK: Reaction 6 negative control, R7NK: Reaction 7 negative control, R8NK: Reaction 8 negative control, R9NK: Reaction 9 negative control, R10NK: Reaction 10 negative control, R11NK: Reaction 11 negative control, PK: Reaction 1 TFPI2 region positive control. This sequence (S1, S2, S3, etc.) can be increased depending on the number of samples. One NK is sufficient for each reaction, regardless of the number of samples.

**Table 1**

| | 1 | 2 | 3 | 4 | 5 | 6 | 7 | 8 | 9 | 10 | 11 | 12 |
|---|---|---|---|---|---|---|---|---|---|---|---|---|
| A | S1-G-R1 | S1-G-R1 | S1-G-R1 | S1-G-R1 | S1-G-R1 | S1-G-R1 | S1-G-R1 | S1-G-R1 | S1-G-R1 | S1-G-R1 | S1-G-R1 | |
| B | S1-S-R1 | S1-S-R2 | S1-S-R3 | S1-S-R4 | S1-S-R5 | S1-S-R6 | | | | | | |
| C | RINK | R2NK | R3NK | R4NK | R5NK | R6NK | R7NK | R8NK | R9NK | R10NK | R11NK | PK |
| D | | | | | | | | | | | | |
| E | | | | | | | | | | | | |
| F | | | | | | | | | | | | |
| G | | | | | | | | | | | | |
| H | | | | | | | | | | | | |

### Reaction preparation for each sample:

The reaction mixtures to be prepared in multiplex for each sample are given in Table 2.

**Table 2**

| | **Volume to be added for a 20 µL reaction** |
|---|---|
| 2X Reaction Mix | 10 µL |
| Bisulfidized Sample DNA | 4 µL |
| PCR Grade Water | 3 µL |
| Reaction Primer-Probe Mix | 3 µL |
| **TOTAL VOLUME** | **20 µL** |

| | |
|---|---|
| Mixes are prepared for each sample according to Table 2. | |

Each target region has its own primer-probe mix.

A Negative Control is added instead of DNA to the NK wells.

A Positive Control containing the TFPI2 gene region is added to the PK wells.

The tube/plate is closed with an optically transparent material (lid/seal).

If a strip tube is used, a spin-down is performed; if a 96-well plate is used, a brief centrifugation (30 seconds, 500 g, using a plate rotor) is performed.

The reaction protocol is created in the qPCR device software according to the following steps:

| | |
|---|---|
| 95°C - 5 min | 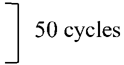 |
| 95°C - 10 sec | |
| 60°C - 30 sec | |

The prepared strip tube/plate is placed in the qPCR device and the reaction is initiated.

### Result Interpretation:

For each run, a negative result must be obtained from the negative control (NK) and a positive result must be obtained from the positive control (PK) for the relevant probe. Because the probes are run individually for high sensitivity, they should give a signal with a corresponding single dye. Probes are stained with FAM or HEX. Amplification should be observed for the FAM-labeled probe in the PK well, but not for the HEX-labeled probe. Amplification should not be observed for the FAM- and HEX-labeled probe in the NK well. Each reaction represents a target gene region.

**Table 4. Evaluation of qPCR results.**

| **Stool Reaction** | **Target Region** | **Stool Reaction** | **Target Region** | **Serum Reaction** | **Target Region** |
|---|---|---|---|---|---|
| Reaction 1 | TFPI2 | Reaction 7 | ADHFE1 | Reaction 1 | TFPI2 |
| Reaction 2 | NDRG4 | Reaction 8 | HOXA2 | Reaction 2 | SEPTIN9 |
| Reaction 3 | SEPTIN9 | Reaction 9 | BMP3 | Reaction 3 | SDC2 |
| Reaction 4 | ITGA4 | Reaction 10 | MGMT | Reaction 4 | HOXA2 |
| Reaction 5 | SDC2 | Reaction 11 | BMP3 - Metile olmayan | Reaction 5 | SFRP2 |
| Reaction 6 | SFRP2 | Positive Control | TFPI2 (Methylated) | Reaction 6 | BMP3 - Unmethylated |

Specificity analyses were performed for each reaction. Signaling sensitivity in colorectal cancer cell lines and clinical cancer samples, and specificity in control samples were calculated.

LOD (Limit of Detection) studies were also conducted for each target region. LOD values were shown to be between 0.2 and 2 ng for the HT29 and CaCo-2 cancer cell line DNAs used as positive controls.

The minimum requirements for the test to be considered positive are as follows: in stool samples, (TFPI2 and SEPTIN9) or ITGA4 or SDC2, or MGMT must be positive, or in serum specimens (TFPI2 and SFRP2), or SDC2 must be positive. Positivity for other genes increases the test score, increasing the likelihood that the sample originates from a cancer patient. The qPCR amplification results of the 5-gene test performed using stool samples from 5 CRC patients using the methylation-based qPCR kit for early diagnosis of CRC, which is the outcome of the invention, are shown in Figure 1.

The qPCR amplification results of the second group of 5-gene test performed using stool samples from 5 CRC patients using the methylation-based qPCR kit for early diagnosis of CRC, which is the outcome of the invention, are shown in Figure 2.

The qPCR amplification results of a 5-gene serum test performed using a methylation-based qPCR kit using serum sprecimens from three CRC patients are shown in Figure 3.

The qPCR amplification results of a test performed using a methylation-based qPCR kit for early diagnosis of CRC, using serum samples from 23 control subjects and two CRC patients, are shown in Figure 4.

The ROC curve, sensitivity, and specificity results generated from the ROC analysis performed after analyzing stool and serum samples from 48 CRC patients and 33 healthy controls are shown in Figure 5.

## Claims

1. Real-time polymerase chain reaction (qPCR) was performed simultaneously for 10 target genes in stool samples and 5 target genes in serum samples from the same patient.
Using specific primers and probe sets labeled with fluorophores for each target gene region, the methylation status of methylated CpG islands belonging to the BMP3, NDRG4, SEPTIN9, ITGA4, SFRP2, SDC2, MGMT, TFPI2, ADHFE1, and HOXA2 genes and the unmethylated CpG island of BMP3 as a control was identified in 11 single reactions for 10 target gene regions in the stool samples.
The methylated CpG islands belonging to the SEPTIN9, SFRP2, SDC2, TFPI2, and HOXA2 genes and the unmethylated CpG island of BMP3 as a control was identified in 6 single reactions for 5 target gene regions in the serum specimens. It is a "Quantitative Real-Time Polymerase Chain Reaction Kit Using Primer and Probe Sets for the Simultaneous Methylation Detection of 10 Different Target Gene CpG Sites in Stool and Serum Specimens for the Early Diagnosis of Colorectal Cancer," and, its features include the following in its content and analysis method:

2. The "Quantitative Real-Time Polymerase Chain Reaction Kit Using Primer and Probe Sets for the Simultaneous Methylation Detection of 10 Different Target Gene CpG Sites in Stool and Serum Samples for the Early Diagnosis of Colorectal Cancer," as mentioned in claim 1. It contains 11 primer-probe mixes (1-BMP3, 2-NDRG4, 3-SEPTIN9, 4-ITGA4, 5-SFRP2, 6-SDC2, 7-MGMT, 8-TFPI2, 9-ADHFE1, 10-HOXA2, 11-BMP3-Unmethylated) unique to this kit in 11 brown vials for stool samples, and 6 primer-probe mixes (1-SEPTIN9, 2-SFRP2, 3-SDC2, 4-TFPI2, 5-HOXA2, 6-BMP3-Unmethylated) unique to this kit in 6 brown vials for serum specimens.

3. The "Quantitative Real-Time Polymerase Chain Reaction Kit Using Primer and Probe Sets for the Simultaneous Detection of 10 Different Target Gene CpG Sites in Stool and Serum Samples for the Early Diagnosis of Colorectal Cancer," as mentioned in claim 1, is **characterized by** the following steps in the analysis of the results. For a sample to be considered positive, it must be positive for a combination of five genes (TFPI2 and SEPTIN9) or ITGA4 or SDC2, or MGMT in stool samples, or positive for a combination of three genes (TFPI2 and SFRP2), or SDC2 in serum specimens. Positivity for other genes increases the test score, increasing the likelihood that the test sample originates from a cancer patient.
